# EUROPÄISCHE PATENTANMELDUNG

(11) **EP 1 938 785 A2**
(43) Veröffentlichungstag der Anmeldung: **02.07.2008**
(21) Anmeldenummer: 07023931.4
(22) Anmeldetag: 11.12.2007
(51) Int. Cl.: A61K 8/06, A61K 8/34, A61K 8/37, A61K 8/60, A61Q 19/00, A61P 17/10

(54) **Kosmetische und dermatologische Öl-in-Wasser-Emulsionen mit selbstkonservierenden Eigenschaften**

(30) Priorität: 28.12.2006 DE 102006062501
(71) Anmelder: Henkel AG & Co. KGaA, 40589 Düsseldorf (DE)
(72) Erfinder: Anderheggen, Bernd, 42781 Haan (DE); Janssen, Frank, 41470 Neuss (DE)

(57) **Zusammenfassung**

Die Anmeldung betrifft kosmetische und dermatologische Öl-in-Wasser-Emulsionen, die ausgewählte Emulgatoren, Fettsäurealkylester, Glycole und Verdickungsmittel, gegebenenfalls zusammen mit Antioxidantien, in einem ausgewogenen Gewichtsverhältnis zueinander enthalten, so dass sie auch ohne den Zusatz üblicher Konservierungsmittel eine ausreichende Konservierung, sozusagen "selbstkonservierende Eigenschaften", aufweisen.

## Beschreibung

### Eigenschaften

Die Erfindung betrifft kosmetische und dermatologische Öl-in-Wasser-Emulsionen, die ausgewählte Emulgatoren, Fettsäurealkylester, Glycole und Verdickungsmittel, gegebenenfalls zusammen mit Antioxidantien, in einem ausgewogenen Gewichtsverhältnis zueinander enthalten, so dass sie auch ohne den Zusatz üblicher Konservierungsmittel eine ausreichende Konservierung, sozusagen "selbstkonservierende Eigenschaften", aufweisen.

Kosmetische und dermatologische Zusammensetzungen müssen, insbesondere, wenn sie Wasser enthalten, gegenüber der Einwirkung von Mikroorganismen durch den Zusatz von Konservierungsmitteln haltbar gemacht werden. Sie werden in niedrigen Konzentrationen - meist unter 0,5 Gew.-% - zugesetzt, um einen mikrobiell bedingten Verderb zu verzögern. Im Unterschied zu den Desinfektionsmitteln und Begasungsmitteln, die gegen Mikroorganismen rasch abtötend (mikrobizid) wirken und zumeist toxisch sind, hemmen die Konservierungsmittel über einen längeren Zeitraum lediglich Wachstum und Vermehrung der Mikroorganismen, d.h. sie wirken in den üblicherweise angewandten Konzentrationen eher mikrobiostatisch. Es kann jedoch auch ein Konservierungsmittel bei ausreichender Dosierung die vorhandenen Mikroorganismen unter Umständen innerhalb weniger Tage abtöten, wenn der Keimgehalt im Kosmetikum klein und eine nachträgliche Kontamination mit Mikroorganismen ausgeschlossen ist. Das Auskeimen von Bakteriensporen und die Bildung von Mykotoxinen durch Schimmelpilze können durch Konservierungsmittel ebenfalls unterdrückt werden. Die meisten Konservierungsmittel sind vor allem gegen Hefen und Schimmelpilze wirksam, nur wenige lassen sich wirkungsvoll gegen Bakterien einsetzen.
Da die meisten Konservierungsmittel schwach sauer reagieren und nur neutrale, undissoziierte Moleküle die Zellmembran passieren und in das Zellinnere der Mikroben gelangen können, sind diese Konservierungsmittel nur in stärker sauren Zusammensetzungen verwendbar. Je niedriger der pH-Wert in einem solchen Kosmetikum und je kleiner die stoffspezifische Dissoziationskonstante des jeweiligen Konservierungsmittels ist, umso größer ist der undissoziierte, antimikrobiell wirksame Anteil des Konservierungsmittels.
Trotz der hohen Zahl der zur Konservierung von kosmetischen Mitteln zugelassenen Stoffe haben in der Praxis nur wenige Verbindungen eine größere Bedeutung bei der Konservierung von kosmetischen Mitteln. In den USA sind dies z. B. 4-Hydroxybenzoesäureester (Methylester und Propylester, aber auch die Ethyl- und Butylester), 1,1'-Methylen-3,3'-bis(1-hydroxymethyl-2,5-dioxo-4-imidazolidinyl)-harnstoff (Germall 115), Kathon CG^{®} (Methylsiothiazolone), DMDM-Hydantoin, Formaldehyd, 2-Phenoxyethanol und Sorbinsäure.
Die oben genannten Alkylester der para-Hydroxybenzoesäure sind auch unter der Kurzbezeichnung "Parabene" bekannt. Neben einem breiteren antimikrobiellen Wirkungsspektrum weisen Parabene als nicht dissoziierende Verbindungen gegenüber den Konservierungssäuren (wie Soebinsäure) den Vorteil auf, auch in höheren pH-Bereichen (bis pH 7,0) noch wirksam zu sein. Auch Kombinationen von Hydroxybenzoesäureestern mit Benzoesäure oder Sorbinsäure sind üblich. Neben der ungünstigen Verteilung der Hydroxybenzoesäureester in Emulsionen ist ein begrenzender Faktor für ihre Anwendung, dass sie infolge ihrer phenolischen Hydroxy-Gruppe von Eiweißen, Emulgatoren (z.B. Lecithin) und anderen Substratbestandteilen zum Teil inaktiviert werden. Für die Summe aller Ethyl-, Methyl- und Propylester der *p*-Hydroxybenzoesäure und ihrer Natriumsalze *(Parabene)* wurde ein temporärer ADI-Wert (acceptable daily intake) von 0 - 10 mg/kg festgelegt. *p*-Hydroxybenzoesäureester werden rasch absorbiert und die Ester-Bindung hydrolysiert. Die renale Ausscheidung erfolgt nicht nur als freie *p*-Hydroxybenzoesäure, sondern auch als deren Glucuronide, Sulfat-Konjungate und als *p*-Hydroxyhippursäure. *p*-Hydroxybenzoesäureester erwiesen sich als nicht mutagen, carcinogen und teratogen. Sie können aber bei entsprechend prädisponierten Personen sowohl pseudo-allergische Reaktionen als auch Allergien auslösen.
Obwohl Konservierungsmittel für die meisten kosmetischen Zubereitungen unverzichtbar sind, ist man bei der Rezepturentwicklung darauf bedacht, die benötigte Menge an Konservierungsmitteln möglichst gering zu halten und beispielsweise einen höheren Gehalt an einwertigen Alkoholen wie Ethanol einzuarbeiten, da Ethanol selbst in Mengen ab ca. 10 Gew.-% eine konservierende Wirkung hat. Ethanol kann aber in solchen höheren Mengen irritierend wirken und ist daher nicht für alle Anwendungsbereiche der Kosmetik geeignet.
Öl-in-Wasser-Emulsionen genießen aufgrund ihres angenehm leichten, nicht-fettenden Hautgefühls beim Verbraucher und aufgrund der mit dem hohen Wassergehalt verbundenen ökonomischen Vorteile auch beim Hersteller hohe Beliebtheit. Da für einen mikrobiellen Verderb vor allem der Wassergehalt verantwortlich ist, ist es für den Kosmetikfachmann eine ständige Herausforderung, die Lagerstabilität zu maximieren und gleichzeitig die Einsatzmenge an Konservierungsmitteln zu minimieren.
Es besteht daher ein ständiger Bedarf an kosmetischen oder dermatologischen Rezepturen, deren Bestandteile mit einem Minimum an eigentlichen Konservierungsmitteln auskommen und dennoch eine ausreichende Produktstabilisierung gewährleisten.

Eine Aufgabe der vorliegenden Anmeldung war es, kosmetische oder dermatologische Rezepturen auf der Basis von Öl-in-Wasser-Emulsionen zu entwickeln, die mit einem möglichst geringen Anteil an Konservierungsmitteln gegenüber mikrobiellem Verderb geschützt werden können. Eine weitere Aufgabe der vorliegenden Anmeldung war es, kosmetische oder dermatologische Rezepturen auf der Basis von Öl-in-Wasser-Emulsionen zu entwickeln, die auch ohne Konservierungsmittel gegenüber mikrobiellem Verderb geschützt werden können.

Überraschend wurde gefunden, dass sich mit einer ausgewogenen Kombination ausgewählter Emulgatoren, Fettsäurealkylester, Polyole und Verdickungsmittel, gegebenenfalls zusammen mit Antioxidatien, konservierungsmittelfreie Öl-in-Wasser-Emulsionen herstellen lassen, die auch ohne Konservierungsmittel gegenüber mikrobiellem Verderb in für kosmetische oder dermatologische Produkte ausreichendem Maß stabil sind.

Gegenstand der vorliegenden Erfindung sind kosmetische oder dermatologische Öl-in-Wasser-Emulsionen zur topischen Behandlung der Haut, enthaltend
a) mindestens einen linearen, gesättigten primären Alkohol mit 14 - 30 Kohlenstoffatomen,
b) mindestens ein Alkylglycosid mit einem linearen oder verzweigten Alkyl- oder Alkenylrest mit 14 - 30 Kohlenstoffatomen und einem Mono-, Di- oder Triglycosidrest,
c) mindestens einen Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einen Ester aus Pentaerythrit und einer linearen oder verzweigten, gesättigten oder ungesättigten Fettsäure mit 3 - 30 Kohlenstoffatomen,
e) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%): (0,1 - 2 Gew.-%) : (0,5 - 5 Gew.-%) : (1 - 7 Gew.-%) : (1 - 25 Gew.-%) : (0,1 - 1 Gew.-%) vorliegen, jeweils bezogen auf das Gesamtgewicht der kosmetischen oder dermatologischen Öl-in-Wasser-Emulsion.

Ein weiterer Gegenstand der vorliegenden Erfindung ist die Verwendung von
a) mindestens einem linearen, gesättigten primären Alkohol mit 14 - 30 Kohlenstoffatomen,
b) mindestens einem Alkylglycosid mit einem linearen oder verzweigten Alkyl- oder Alkenylrest mit 14 - 30 Kohlenstoffatomen und einem Mono-, Di- oder Triglycosidrest,
c) mindestens einem Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einem Ester aus Pentaerythrit und einer linearen oder verzweigten, gesättigten oder ungesättigten Fettsäure mit 3 - 30 Kohlenstoffatomen,
e) mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%) : (0,1 - 2 Gew.-%) : (0,5 - 5 Gew.-%) : (1 - 7 Gew.-%): (1 - 25 Gew.-%): (0,1 - 1 Gew.-%) vorliegen, jeweils bezogen auf das Gesamtgewicht der kosmetischen oder dermatologischen Öl-in-Wasser-Emulsion, zur Herstellung einer konservierungsmittelfreien kosmetischen oder dermatologischen Öl-in-Wasser-Emulsion.

Ein weiterer Gegenstand der vorliegenden Erfindung ist die Verwendung von
a) mindestens einem linearen, gesättigten primären Alkohol mit 14 - 30 Kohlenstoffatomen,
b) mindestens einem Alkylglycosid mit einem linearen oder verzweigten Alkyl- oder Alkenylrest mit 14 - 30 Kohlenstoffatomen und einem Mono-, Di- oder Triglycosidrest,
c) mindestens einem Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einem Ester aus Pentaerythrit und einer linearen oder verzweigten, gesättigten oder ungesättigten Fettsäure mit 3 - 30 Kohlenstoffatomen,
e) mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%) : (0,1 - 2 Gew.-%) : (0,5 - 5 Gew.-%) : (1 - 7 Gew.-%) : (1 - 25 Gew.-%) : (0,1 - 1 Gew.-%), jeweils bezogen auf das Gesamtgewicht der kosmetischen oder dermatologischen Öl-in-Wasser-Emulsion, vorliegen, zur Herstellung einer kosmetischen oder dermatologischen Öl-in-Wasser-Emulsion, die das Wachstum von Propionibacterium acnes inhibiert und/oder zur Behandlung von unreiner Haut und/oder Akne.

Erfindungsgemäß bevorzugte kosmetische oder dermatologische Zusammensetzungen sind dadurch gekennzeichnet, dass sie mindestens ein Antioxidans enthalten.
Erfindungsgemäß bevorzugte Antioxidantien sind ausgewählt ist aus 2,2-Dialkylchromanen, 2,2-Dialkylchromenen, Flavonoiden, Ubichinonen, Ubichinolen, Pentaerythrittetrakis[3(3,5-di-tert.-butyl-4-hydroxyphenyl)propionat], Urocaninsäure, Carotinoiden, Ascorbinsäure und ihren Derivaten, insbesondere ihren Estern und Salzen, Isoascorbinsäure und ihren Derivaten, insbesondere ihren Estern und Salzen, Ferulasäure und ihren Estern, insbesondere Ethylferulat, Kaffeesäure, Rosmarinsäure, 2,6-Di-tert.-butyl-4-methylphenol, tert.-Butyl-4-methoxyphenol, sowie Mischungen hiervon.
Bekannte und erfindungsgemäß bevorzugte 2,2-Dialkylchromane sind die Tocopherole und Tocotrienole. Die Tocopherole sind in 2-Position mit einem Methylrest und mit einem gesättigten 4',8',12'-Trimethyltridecyl-Rest substituiert und weisen in 5-, 7- und 8-Position entweder ein Wasserstoffatom oder eine Methylgruppe auf (α-, β-, γ-, δ-, ζ₂- und η-Tocopherole). Die Tocotrienole, auch als ε-Tocopherole bezeichnet, unterscheiden sich von den vorgenannten Tocopherolen dadurch, dass in 2-Position an Stelle des gesättigten 4',8',12'-Trimethyltridecyl-Restes ein dreifach ungesättigter 4',8',12'-Trimethyltridecatrienyl-Rest aus drei Isoprenyl-Einheiten vorhanden ist. Neben ihrer Anwendung als Vitamin E wirken Tocopherole als Antioxidantien in Fetten und Ölen. Darüber hinaus besitzen Tocopherole eine Vielzahl von günstigen physiologischen Eigenschaften, z. B. Reduzierung von Muskelschäden, die auf oxidativen Streß während körperlicher Höchstleistung zurückzuführen sind, Verminderung des Risikos der Kataraktbildung, Verminderung des oxidativen Stresses bei Rauchern sowie anticarcinogene Effekte. Kosmetisch zeigen sie eine protektive Wirkung gegen Hautschäden und Hautalterung und schützen das Haar vor Witterungseinflüssen.
Neben den erwähnten Tocopherolen und Tocotrienolen sind weitere 2,2-Dialkylchroman- Derivate als kosmetische Wirkstoffe bekannt und erfindungsgemäß ebenfalls bevorzugt. EP 1 174 140 A1 offenbart Chromanolglycoside als Wirkstoff, der effektiv aktiven Sauerstoff und freie Radikale eliminiert. Explizit offenbart ist 2,5,7,8-Tetramethylchroman-6-ol, das in 2-Position mit einem Glucopyranosylmethyl-, Galactopyranosylmethyl-, Fructofuranosylmethyl- oder Mannopyranosylmethyl-Rest substituiert ist. US 5,811,083 offenbart das Amid aus 3,4-Dihydro-6-hydroxy-2,5,7,8-tetramethyl-1-benzopyran-2-carbonsäure (Trolox C) und Cysteamin als Antioxidans gegen Lipidperoxidation. EP 655 239 A1 offenbart die Verwendung von 2,2-Dimethylchromanen und -chromenen, die in 6- und 7-Position mit OH-, CH₃O- oder CF₃CH₂O-Gruppen substituiert sein können, als Antioxidantien in Doxorubicin-haltigen Liposomen. Daran anknüpfend, offenbart EP 1 002 533 A1 die Verwendung der genannten 2,2-Dimethylchromane und -chromene als Antioxidantien in Zusammensetzungen zur topischen oder oralen Verabreichung. Besonders bevorzugte 2,2-Dimethylchromane und -chromene sind 6,7-disubstituierte 2,2-Dialkylchromane oder -chromene der allgemeinen Formeln (CHROM-I) oder (CHROM-II), 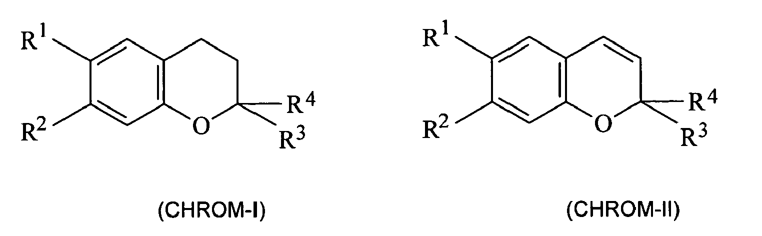 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen.

Erfindungsgemäß besonders bevorzugte kosmetische oder dermatologische Zusammensetzungen sind dadurch gekennzeichnet, dass sie mindestens ein Antioxidans vom Typ der 2,2-Dialkylchromane oder -chromene, außerordentlich bevorzugt mindestens ein 6,7-disubstituiertes 2,2-Dialkylchroman oder -chromen der allgemeinen Formeln (CHROM-I) oder (CHROM-II) enthalten.

Erfindungsgemäß außerordentlich bevorzugte Antioxidantien vom Typ der 6,7-disubstituierten 2,2-Dialkylchromane oder -chromene der allgemeinen Formeln (CHROM-I) oder (CHROM-II) sind ausgewählt aus 2,2-Dimethyl-6-hydroxy-7-methoxy-chroman mit der systematischen Bezeichnung 3,4-Dihydro-7-methoxy-2,2-dimethyl-2H-1-benzopyran-6-ol, das unter der Bezeichnung Lipochroman-6 (INCI: DIMETHYLMETHOXY CHROMANOL) von der Firma Lipotec erhältlich ist.
Besonders bevorzugt wird DIMETHYLMETHOXY CHROMANOL in Mengen von 0,001 - 0,05 Gew-.-%, außerordentlich bevorzugt 0,01 - 0,03 Gew.-%, jeweils bezogen auf das Gewicht der erfinudngsgemäßen Öl-in-Wasser-Emulsion, eingesetzt.

Die erfindungsgemäß bevorzugten Antioxidantien vom Typ der Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Penta-hydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Mono-xerutin (3,3',4',5-Tetrahydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß bevorzugt werden die Flavonoide in Mengen von 0,0001 bis 1 Gew.-%, besonders bevorzugt 0,0005 bis 0,5 Gew.-% und außerordentlich bevorzugt 0,001 bis 0,1 Gew.-%, jeweils bezogen auf die Flavonoidaktivsubstanz in der gesamten kosmetischen Zusammensetzung, eingesetzt.

Erfindungsgemäß bevorzugte Antioxidantien vom Typ der Ubichinone und Ubichinole oder deren Derivate sind im folgenden dargestellt. Ubichinole sind die reduzierte Form der Ubichinone. Die erfindungsgemäß bevorzugten Ubichinone weisen die Formel (UBI-I) auf: 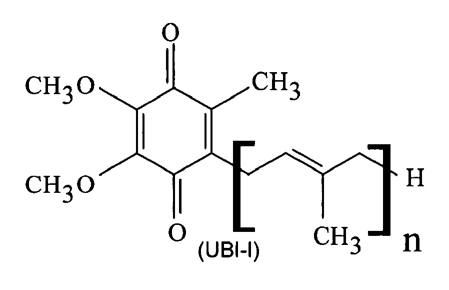 mit n = 6, 7, 8, 9 oder 10.
Besonders bevorzugt ist das Ubichinon der Formel (UBI-I) mit n = 10, auch bekannt als Coenzym Q10.
Erfindungsgemäß bevorzugt werden die Ubichinone, Ubichinole oder deren Derivate in einer Gesamtmenge von 0,0001 - 1 Gew.-%, besonders bevorzugt 0,001 - 0,5 Gew.-% und außerordentlich bevorzugt 0,005 - 0,1 Gew.-%, jeweils bezogen auf die gesamte erfindungsgemäße Zusammensetzung, eingesetzt.

Weitere erfindungsgemäß bevorzugte kosmetische oder dermatologische Zusammensetzungen sind dadurch gekennzeichnet, dass sie insgesamt, jeweils bezogen auf das Gesamtgewicht der Öl-in-Wasser-Emulsion, mindestens ein Antioxidans in einer Gesamtmenge von 0,001 - 1 Gew.-%, bevorzugt 0,005 - 0,5 Gew.-%, besonders bevorzugt 0,01 - 0,3 Gew.-% und außerordentlich bevorzugt 0,05 - 0,1 Gew.-%, enthalten.

Weitere erfindungsgemäß bevorzugte kosmetische oder dermatologische Zusammensetzungen sind dadurch gekennzeichnet, dass sie insgesamt, jeweils bezogen auf das Gesamtgewicht der Öl-in-Wasser-Emulsion, maximal 0,3 Gew.-%, bevorzugt maximal 0,2 Gew.-% und besonders bevorzugt maximal 0,1 Gew.-% an Konservierungsmitteln enthalten.
Weitere erfindungsgemäß bevorzugte kosmetische oder dermatologische Zusammensetzungen sind dadurch gekennzeichnet, dass sie frei sind von Konservierungsmitteln.
Unter Konservierungsmitteln sind im Sinne der vorliegenden Erfindung alle Verbindungen zu verstehen, die gemäß CTFA als Konservierungsmittel ausgewiesen sind, insbesondere folgende Verbindungen: Formaldehyd, Formaldehydabspalter (wie z. B. 1,3-Dimethylol-4,4-dimethylhydantoin, INCI-Bezeichnung DMDM Hydantoin, z. B. unter der Handelsbezeichnung Glydant von der Firma Lonza erhältlich), lodopropylbutylcarbamate wie 3-lod-2-propinylbutylcarbamat (z. B. die unter den Handelsbezeichnungen Glycacil-L, Glycacil-S von der Firma Lonza erhältlichen und/oder Dekaben LMB von Jan Dekker), Parabene (d. h. p- Hydroxybenzoesäurealkylester, wie Methyl-, Ethyl-, Propyl- und/oder Butylparaben), Phenoxyethanol, Benzoesäure, Dibromdicyanobutan (2-Brom-2-brommethylglutarodinitril), 2-Brom-2-nitro-propan-1,3-diol, Imidazolidinylharnstoff und dessen Derivate, insbesondere 1,1'-Methylen-3,3'-bis(1-hydroxymethyl-2,5-dioxo-4-imidazolidinyl)-harnstoff (z. B. Germall 115), Methylsiothiazolone, insbesondere 5-Chlor-2-methyl-4-isothiazolin-3-on, 2-Chloracetamid, Benzalkoniumchlorid, Benzylalkohol, Sorbinsäure, ihre Salze und ihre Ester, sowie Salicylsäure, ihre Salze und ihre Ester.

Als eine Hauptkomponente enthalten die erfindungsgemäßen Zusammensetzungen mindestens einen linearen, gesättigten primären Alkohol mit 14 - 30 Kohlenstoffatomen in einer Gesamtmenge von 1,5 - 14 Gew.-%, bevorzugt in einer Gesamtmenge von 3 - 10 Gew.-%, besonders bevorzugt 5 - 8,5 Gew.-% und außerordentlich bevorzugt 7 - 8 Gew.-%, jeweils bezogen auf das Gesamtgewicht der kosmetischen oder dermatologischen Öl-in-Wasser-Emulsion.
Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol, Behenylalkohol, Lignocerylalkohol, Cerylalkohol und Myricylalkohol und Mischungen hiervon, insbesondere technische Mischungen wie Arachidylalkohol und Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon.

Als eine weitere Hauptkomponente enthalten die erfindungsgemäßen Zusammensetzungen mindestens ein Alkylglycosid mit einem linearen oder verzweigten Alkyl- oder Alkenylrest mit 14 - 30 Kohlenstoffatomen und einem Mono-, Di- oder Triglycosidrest in einer Gesamtmenge von 0,1 - 2 Gew.-%, bevorzugt 0,3 - 1 Gew.-%, besonders bevorzugt 0,4 - 0,8 Gew.-% und außerordentlich bevorzugt 0,6 - 0,7 Gew.-%, jeweils bezogen auf das Gesamtgewicht der kosmetischen oder dermatologischen Öl-in-Wasser-Emulsion.
Die auch als Zuckertenside vom Typ der Alkyl- und Alkenyloligoglycoside bezeichneten Komponenten weisen die allgemeine Formel (E4-II) auf,

R⁴O-[G]ₚ (E4-II)

in der R⁴ für einen Alkyl- oder Alkenylrest mit 14 - 30, bevorzugt 16 - 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglycoside können sich von Aldosen bzw. Ketosen mit 5 oder 6 Kohlenstoffatomen, wie insbesondere Fructose, Glucose und Xylose, vorzugsweise von Glucose (sogenannte APG) oder Xylose (sogenannte APX), ableiten, wobei Glucose als Zuckerrest außerordentlich bevorzugt ist. Die bevorzugten Alkyl- und/oder Alkenyloligoglycoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglycosiden, an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglycosid eine analytisch ermittelte rechnerische Größe, die meistens eine gebrochene Zahl darstellt. Vorzugsweise werden Alkyl- und/oder Alkenyloligoglycoside mit einem mittleren Oligomerisierungsgrad p von 1,1 bis 3,0 eingesetzt. Aus anwendungstechnischer Sicht sind solche Alkyl- und/oder Alkenyloligoglycoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 14 - 30, bevorzugt 16 - 22 Kohlenstoffatomen ableiten. Typische Beispiele sind Myristylalkohol, Cetylalkohol, Palmitoleylalkohol, Stearylalkohol, lsostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische.

Besonders bevorzugt werden die Alkyl- und Alkenyloligoglycoside als Gemisch mit dem entsprechenden, zur Alkylierung eingesetzten Alkoholschnitt eingesetzt. Hierbei handelt es sich um sogenannte selbstemulgierende Mischungen aus langkettigen Fettalkoholen und entsprechend alkylierten Glycosiden. Bevorzugt werden Mischungen eingesetzt, die unter den Bezeichnungen Montanov 68, Montanov 202, Montanov L und Montanov 82 von der Firma Seppic erhältlich sind. Diese Mischungen enthalten ca. 15 Gew.-% Alkylglucosid und ca. 85 Gew.-% Fettalkohole. Selbstverständlich können die Alkyl- und Alkenyloligoglycoside aber auch in reiner Form eingesetzt werden.

Als eine weitere Hauptkomponente enthalten die erfindungsgemäßen Zusammensetzungen mindestens einen Ester aus einem geradkettigen oder verzweigten Alkanol mit 1 bis 30 Kohlenstoffatomen und einer geradkettigen oder verzweigten Fettsäure mit 3 bis 18 Kohlenstoffatomen in einer Gesamtmenge von 0,5 - 5 Gew.-%, bevorzugt 0,6 - 4 Gew.-%, besonders bevorzugt 1,0 - 3 Gew.-% und außerordentlich bevorzugt 1,5 - 2 Gew.-%, jeweils bezogen auf das Gesamtgewicht der kosmetischen oder dermatologischen Öl-in-Wasser-Emulsion. Es versteht sich von selbst, dass die Komponenten c) und d) voneinander verschieden sind, das heißt, der Alkanolteil des Esters c) ist nicht Pentaerythrit.
Dieser Ester c) dient als Ölkomponente. Erfindungsgemäß bevorzugte Ester sind ausgewählt aus Cetylisononanoat, Stearylisononanoat, Cetearylisononanoat (Cetiol^{®} SN), Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868), Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Als eine weitere Hauptkomponente enthalten die erfindungsgemäßen Zusammensetzungen mindestens einen Ester aus Pentaerythrit und einer linearen oder verzweigten, gesättigten oder ungesättigten Fettsäure mit 3 - 30 Kohlenstoffatomen in einer Gesamtmenge von 1 - 7 Gew.-%, bevorzugt 1,5 - 6 Gew.-%, besonders bevorzugt 2 - 5 Gew.-% und außerordentlich bevorzugt 3 - 4 Gew.-%, jeweils bezogen auf das Gesamtgewicht der kosmetischen oder dermatologischen Öl-in-Wasser-Emulsion. Dieser Ester dient vor allem als Coemulgator. Erfindungsgemäß bevorzugte Pentaerythritester sind ausgewählt aus Pentaerythritylmonostearat, Pentaerythrityldistearat, Pentaerythrityltristearat, Pentaerythrityltetrastearat sowie Mischungen hiervon. Die erfindungsgemäß bevorzugten sind zum Beispiel als Handelsprodukte Cutina^{®} PES (INCI: Pentaerythrityl distearate), erhältlich. Dieses Handelsprodukt stellt eine technische Mischung aus Mono- und Diestern, aber auch Tri- und Tetraestern dar.

Als eine weitere Hauptkomponente enthalten die erfindungsgemäßen Zusammensetzungen mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon in einer Gesamtmenge von 1 - 25 Gew.-%, bevorzugt 7 - 20 Gew.-%, besonders bevorzugt 10 - 17 Gew.-% und außerordentlich bevorzugt 13 - 15 Gew.-%, jeweils bezogen auf das Gesamtgewicht der kosmetischen oder dermatologischen Öl-in-Wasser-Emulsion.
Bevorzugt sind diese Polyolkomponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und Zuckeralkohole wie Fructose, Glucose, Maltose, Maltitol, Mannit, Inosit, Sucrose, Trehalose und Xylose sind erfindungsgemäß geeignet. Besonders bevorzugte Polyolkomponenten sind ausgewählt aus 1,2-Propylenglycol, Glycerin, 1,2-Hexandiol, 1,6-Hexandiol, 1,3-Butylenglycol, Dipropylenglycol, Tripropylenglycol und PEG-8 sowie Mischungen hiervon. Außerordentlich bevorzugt sind 1,2-Propylenglycol, Glycerin, 1,6-Hexandiol und insbesondere Mischungen hiervon, insbesondere Dreiermischungen aus 1,2-Propylenglycol, Glycerin und 1,6-Hexandiol.
Es wurde überraschend festgestellt, dass Mischungen aus 1,2-Propylenglycol, Glycerin und 1,6-Hexandiol hervorragend die selbstkonservierenden Eigenschaften der erfindungsgemäßen Öl-in-Wasser-Emulsionen unterstützen.
Weiterhin wurde überraschend festgestellt, dass Mischungen aus 1,2-Propylenglycol, Glycerin und 1,6-Hexandiol hervorragend die hemmende Wirkung der erfindungsgemäßen Öl-in-Wasser-Emulsionen gegen die Keime der unreinen Haut, insbesondere Propionibacterium acnes, unterstützen.

Als weitere Hauptkomponente enthalten die erfindungsgemäßen Zusammensetzungen Xanthan-Gum in einer Gesamtmenge von 0,1 - 1 Gew.-%, bevorzugt 0,15 - 0,5 Gew.-%, besonders bevorzugt 0,2 - 0,3 Gew.-%, jeweils bezogen auf das Gesamtgewicht der kosmetischen oder dermatologischen Öl-in-Wasser-Emulsion.

Es wurde überraschend festgestellt, dass Xanthan-Gum in Kombination mit den übrigen erfindungsgemäßen Hauptkomponenten hervorragend die selbstkonservierenden Eigenschaften der erfindungsgemäßen Öl-in-Wasser-Emulsionen unterstützt.
Weiterhin wurde überraschend festgestellt, dass Xanthan-Gum in Kombination mit den übrigen erfindungsgemäßen Hauptkomponenten hervorragend die hemmende Wirkung der erfindungsgemäßen Öl-in-Wasser-Emulsionen gegen die Keime der unreinen Haut, insbesondere Propionibacterium acnes, unterstützt.

Besonders bevorzugte erfindungsgemäße Öl-in-Wasser-Emulsionen sind dadurch gekennzeichnet, dass sie 7 - 10 Gew.-% Komponente a), 0,4 - 0,8 Gew.-% Komponente b); 1 - 3 Gew.-% Komponente c), 2 - 4 Gew.-% Komponente d), 10 - 15 Gew.-% Komponente e) und 0,1 - 0,2 Gew.-% Xanthan-Gum, enthalten, jeweils bezogen auf das Gesamtgewicht der Komponente in der gesamten Zusammensetzung.

Weitere besonders bevorzugte erfindungsgemäße Öl-in-Wasser-Emulsionen sind dadurch gekennzeichnet, dass sie 7 - 8,5 Gew.-% Komponente a), 0,4 - 0,8 Gew.-% Komponente b); 0,6 - 4 Gew.-% Komponente c), 2 - 4 Gew.-% Komponente d), 10 - 15 Gew.-% Komponente e) und 0,1 - 0,2 Gew.-% Xanthan-Gum, enthalten, jeweils bezogen auf das Gesamtgewicht der Komponente in der gesamten Zusammensetzung.

Weitere besonders bevorzugte erfindungsgemäße Öl-in-Wasser-Emulsionen sind dadurch gekennzeichnet, dass sie 7 - 8 Gew.-% Komponente a), 0,4 - 0,6 Gew.-% Komponente b); 1 - 3 Gew.-% Komponente c), 2 - 4 Gew.-% Komponente d), 10 - 15 Gew.-% Komponente e) und 0,1 - 0,2 Gew.-% Xanthan-Gum, enthalten, jeweils bezogen auf das Gesamtgewicht der Komponente in der gesamten Zusammensetzung.

Besonders bevorzugte erfindungsgemäße Öl-in-Wasser-Emulsionen sind dadurch gekennzeichnet, dass sie zur Verbesserung der sensorischen Eigenschaften weiterhin mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten vom Typ DRY FLO^{®}, Cellulose und Cellulosederivaten, Siliciumdioxid, Kieselsäuren, z. B. Aerosil^{®}-Typen, sphärischen Polyalkylsesquisiloxan-Partikeln (insbesondere Aerosil^{®} R 972 und Aerosil^{®} 200V von Degussa), Kieselgelen, Talkum, Kaolin, Tonen, z. B. Bentoniten, Magnesiumaluminiumsilikaten, Bornitrid, Lactoglobulinderivaten, z. B. Natrium-C₈₋₁₆-lsoalkyl-succinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.
Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).
Besonders bevorzugte erfindungsgemäße Öl-in-Wasser-Emulsionen sind dadurch gekennzeichnet, dass sie mindestens einen festen, wasserunlöslichen teilchenförmigen Füllstoff in einer Gesamtmenge von 0,01 bis 10 Gew.-%, bevorzugt 0,1 - 5 Gew.-%, besonders bevorzugt 1 - 2 Gew.-%, jeweils bezogen auf die Gesamtzusammensetzung, enthalten.

Weitere bevorzugte Zusatzstoffe sind Verdickungsmittel, besonders bevorzugt natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS, Simulgel^{®} EG (SODIUM ACRYLATE/ SODIUM ACRYLOYLDIMETHYL TAURATE COPOLYMER) und Sepiplus 400 (POLYACRYLATE-13) der Firma SEPPIC sowie Aristoflex AVC (AMMONIUM ACRYLOYLDIMETHYLTAURATE/VINYLPYRROLI-DONE COPOLYMER) der Firma Clariant. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Die selbstkonservierenden Eigenschaften der erfindungsgemäßen Öl-in-Wasser-Emulsionen kann neben der Behandlung unreiner Haut und Akne einer Vielzahl von weiteren Anwendungen zu gute kommen, insbesondere der Tagespflege von alternder Haut oder in der Antiageing-Kosmetik, aber auch für Lichtschutzcremes, Körpercremes, Nachtpflegecremes usw.
Die Wirkung der erfindungsgemäßen Wirkstoffkombination, insbesondere in Bezug auf die Verbesserung der Oberflächenstruktur der Haut, kann weiter gesteigert werden durch mindestens einen Wirkstoff, der die Kollagensynthese stimuliert.
Erfindungsgemäß besonders bevorzugte die Kollagensynthese stimulierende Wirkstoffe sind ausgewählt aus Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist, weiterhin aus den Tripeptiden Gly-His-Lys, Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure) und Dap-Val-Lys, weiterhin aus dem Tetrapeptid Rigin, Rigin-basierten Tetrapeptiden sowie ALAMCAT-Tetrapeptiden, dem Pentapeptid Lys-Thr-Thr-Lys-Ser, den Hexapeptiden Val-Gly-Val-Ala-Pro-Gly, Ala-Asp-Leu-Lys-Pro-Thr (Hexapeptide-3), Hexapeptide-4, Hexapeptide-5, Hexapeptide-6, Hexapeptide-8, Hexapeptide-9 und Hexapeptide-10, weiterhin ausgewählt aus Sojaproteinhydrolysaten mit einem mittleren Molekulargewicht im Bereich von 600 - 1000 Dalton, bevorzugt 800 Dalton, wobei die genannten Peptide in N-acylierter Form vorliegen können, was erfindungsgemäß besonders bevorzugt sein kann, weiterhin ausgewählt aus Retinoiden, Kombucha, Olivenblattextrakten, Oleanolsäure, Oleanol, Extrakten aus Haferkörnern (Avena Sativa (Oat) Kernel Extract) und liposomenverkapselten Grüntee-Extrakten.
Das Tripeptid Gly-His-Lys ist z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich, stellt aber (in acylierter Form) auch einen Bestandteil des Produktes Matrixyl 3000 von Sederma dar. Das Tripeptid Gly-His-Lys kann auch als Kupfersalz (Cu²⁺) eingesetzt werden und ist als solches über ProCyte Corporation zu beziehen. Weiterhin können bevorzugt Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile bevorzugt geeignet. Die erfindungsgemäß besonders bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn oder Citrullin ersetzt. Ein weiteres erfindungsgemäß bevorzugtes Peptid, das die Kollagenynthese stimuliert, ist dementsprechend Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist.

Weitere erfindungsgemäß bevorzugte Tripeptide, die die Kollagenynthese stimulieren, sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure) und Dap-Val-Lys. Ein besonders bevorzugtes Tripeptid ist Palmitoyl-Lys-Val-Lys, z. B. erhältlich von der Firma Pentapharm unter der Bezeichnung SYN^{®}-COLL.

Weitere erfindungsgemäß bevorzugte Tetrapeptide, die die Kollagenynthese stimulieren, sind das Tetrapeptid Rigin und Rigin-basierte Tetrapeptide sowie ALAMCAT-Tetrapeptide. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg (INCI-Bezeichnung: Palmitoyl Tetrapeptide-1), das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapeptide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z. B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (lle). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Weitere erfindungsgemäß bevorzugte Peptide, die die Kollagensynthese stimulieren, sind das Pentapeptid Lys-Thr-Thr-Lys-Ser und seine N-acylierten Derivate, besonders bevorzugt N-Palmitoyl-Lys-Thr-Thr-Lys-Ser, das unter der Bezeichnung Matrixyl von der Firma Sederma erhältlich ist.

Weitere erfindungsgemäß bevorzugte Hexapeptide, die die Kollagensynthese stimulieren, sind das Hexapeptid Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist. Weitere erfindungsgemäß bevorzugte Peptide sind die Hexapeptide und/oder deren N-acylierten Derivate Ala-Asp-Leu-Lys-Pro-Thr (Hexapeptide-3, z. B. Peptide 02 von Vincience), Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec).

Weitere erfindungsgemäß bevorzugte Wirkstoffe, die die Kollagensynthese stimulieren, sind ausgewählt aus Sojaproteinhydrolysaten mit einem mittleren Molekulargewicht im Bereich von 600 - 1000 Dalton, bevorzugt 800 Dalton. Ein derartiges besonders bevorzugtes Sojaproteinhydrolysat ist unter dem Handelsnamen Phytokine von der Firma Coletica erhältlich.

Weitere erfindungsgemäß bevorzugte Wirkstoffe, die die Kollagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-cis-Retinsäure und 13-cis-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin*), monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte Wirkstoffe, die die Kollagensynthese stimulieren, sind ausgewählt aus Kombucha.
Bei Kombucha handelt es sich um ein aus dem ostasiatischen Raum stammendes, etwa 0,5 Vol.-% Alkohol enthaltendes, angenehm säuerliches, leicht süßes, moussierendes Gärungsgetränk auf Basis von Mono- oder Disaccharid-haltigem Tee (Schwarzer Tee, Grüner Tee, Kräutertee oder Früchtetee), das ohne Zusatz von Konservierungsstoffen, Farbstoffen und Aromastoffen hergestellt wird.
Beim "Kombucha-Teepilz" handelt es sich tatsächlich nicht um einen Pilz, sondern um eine Symbiose von Essigsäurebakterien (meistens *Acetobacter xylinum,* aber auch *Acetobacter gluconicum*) mit verschiedenen Hefen *(Saccharomyces* sp., *Torula* sp., *Pichia fermentans*); fakultativ können Milchsäurebakterien vorhanden sein. Aus Gründen der Produktionssicherheit und Produktionsstabilität wird industriell gefertigter Kombucha oft wärmebehandelt.

Unter "Tee" werden erfindungsgemäß wässrige Aufgüsse von Blättern, Knospen und zarten Stielen von Thea sinensis (Camellia sinensis) und Thea assamica, aber auch von diversen anderen Pflanzen verstanden, beispielsweise Linde (Lindenblüten), Malve, Rotbusch, Kamille usw.

Bevorzugte Kombucha ist aus Tee von fermentierten (Schwarztee, Oolong-Tee, Gelber Tee, Pu-Erh-Tee) oder unfermentierten (Grüntee) Blättern von Thea sinensis (Camellia sinensis) oder Thea assamica gewonnen. Weitere bevorzugte Kombucha ist durch Fermentation von (meist mit Saccharose) gezuckertem Tee mit den symbiotischen Mikroorganismen Saccharomyces und Acetobacter xylinum gewonnen. Ein erfindungsgemäß besonders bevorzugtes Kombucha-Produkt ist ein Produkt, das durch Fermentation von mit Saccharose gezuckertem Schwarztee mit den zwei symbiotischen Mikroorganismen Saccharomyces und Acetobacter xylinum gewonnen wurde und beispielsweise unter der INCI-Bezeichnung "Saccharomyces/Xylinum/Black Tea Ferment, Glycerin, Hydroxyethylcellulose" mit der Handelsbezeichnung "Kombuchka" als Glycerin-haltige verdickte Zubereitung von der Firma Sederma erhältlich ist.

Weitere erfindungsgemäß bevorzugte Wirkstoffe, die die Kollagensynthese stimulieren, sind ausgewählt aus Olivenblattextrakten (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Olivenblattextrakte können einen hohen Gehalt an Oleanolsäure und/oder Oleanol aufweisen. Weitere erfindungsgemäß bevorzugte Wirkstoffe, die die Kollagensynthese stimulieren, sind ausgewählt aus Oleanolsäure und Oleanol.

Weitere erfindungsgemäß bevorzugte Wirkstoffe, die die Kollagensynthese stimulieren, sind ausgewählt aus Extrakten aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich.

Weitere erfindungsgemäß bevorzugte Wirkstoffe, die die Kollagensynthese stimulieren, sind ausgewählt aus liposomenverkapselten Grüntee-Extrakten (Camellia Sinensis). Ein erfindungsgemäß besonders bevorzugter liposomenverkapselter Grüntee-Extrakt ist unter der Handelsbezeichnung Greenselect Phytosome von der Firma Indena erhältlich.

Erfindungsgemäß besonders bevorzugte kosmetische oder dermatologische Zusammensetzungen sind dadurch gekennzeichnet, dass sie neben der erfindungsgemäßen Wirkstoffkombination mindestens einen Wirkstoff, der die Kollagensynthese stimuliert, in einer Gesamtmenge von 0,000001
- 5 Gew.-%, bevorzugt 0,00001 - 2 Gew.-%, besonders bevorzugt 0,0001 - 1 Gew.-% und außerordentlich bevorzugt 0,005 - 0,5 Gew.-%, enthalten, jeweils bezogen auf den Gehalt an Aktivsubstanz in der gesamten Zusammensetzung.

Die Wirkung der erfindungsgemäßen Wirkstoffkombination, insbesondere in Bezug auf die Verbesserung der Elastizität und/oder des Feuchtigkeitsgehaltes der Haut, kann weiter gesteigert werden durch mindestens einen Wirkstoff, der die Interaktion zwischen der extrazellulären Matrix und den Fibroblasten erhöht und/oder verbessert. Weitere erfindungsgemäß bevorzugte kosmetische oder dermatologische Zusammensetzungen sind dadurch gekennzeichnet, dass sie mindestens einen Wirkstoff enthalten, der die Interaktion zwischen der extrazellulären Matrix und den Fibroblasten erhöht und/oder verbessert und der ausgewählt ist aus
- Sojaproteinhydrolysaten mit einem mittleren Molekulargewicht im Bereich von 1200 - 1800 Dalton, bevorzugt im Bereich von 1400 - 1700 Dalton,
- mit Kokosfettsäuren N-acylierten und/oder veresterten Sojaproteinhydrolysaten in Form ihrer Alkalimetallsalze, bevorzugt in Form der Kaliumsalze,
- Ascorbinsäure, den Estern der Ascorbinsäure mit anorganischen und/oder organischen Säuren, den Ethern der Ascorbinsäure mit Mono-, Oligo- und Polysacchariden sowie den physiologisch verträglichen Salzen dieser Komponenten,
- Apfelkernextrakten (Pyrus Malus (Apple) Fruit Extract),
- Lotuskeim-Extrakten (Nelumbo Nucifera Germ Extract),
- Extrakten aus Maiskörnern (Zea Mays (Corn) Kernel Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Hydroxystilbenen und deren Estern, insbesondere Resveratrol und/oder Resveratrolmono-,
- di- und -triphosphorsäureestern und deren Salzen,
- Keratinhydrolysaten, insbesondere Wollkeratinhydrolysaten,
- Conchiolinhydrolysaten,
- dem Dipeptid L-Citrullyl-L-arginin und seinen mit mindestens einer, bevorzugt linearen, C₂-C₂₂-Fettsäure N-acylierten und/oder veresterten Derivaten, bevorzugt Acetyl Citrull Amido Arginine,
- Carnitin,
- Hypotaurin,
- L-Glutamylaminoethyl-indol,
- Isoflavonoiden und Isoflavonoid-reichen Pflanzenextrakten,
- Dihydroquercetin (= Taxifolin),
- sowie Mischungen hiervon.

Erfindungsgemäß bevorzugte Wirkstoffe, die die Interaktion zwischen der extrazellulären Matrix und den Fibroblasten erhöhen und/oder verbessern, sind ausgewählt aus Sojaproteinhydrolysaten mit einem mittleren Molekulargewicht (Mw) im Bereich von 1200 - 1800 Dalton, bevorzugt im Bereich von 1400 - 1700 Dalton (Da). Ein derartiges besonders bevorzugtes Sojaproteinhydrolysat ist unter dem Handelsnamen Ridulisse C von der Firma Silab erhältlich. Ridulisse C enthält vier Molekulargewichtsfraktionen: 0,8 Gew.-% mit einem Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.

Weitere erfindungsgemäß bevorzugte Wirkstoffe, die die Interaktion zwischen der extrazellulären Matrix und den Fibroblasten erhöhen und/oder verbessern, sind ausgewählt aus mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 -18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein erfindungsgemäß besonders bevorzugtes, mit Kokosfettsäuren N-acyliertes und/oder verestertes Sojaproteinhydrolysat in Form des Kaliumsalzes ist unter der Handelsbezeichnung Coccopolipeptide di Soja von der Firma Sinerga erhältlich

Weitere erfindungsgemäß bevorzugte Wirkstoffe, die die Interaktion zwischen der extrazellulären Matrix und den Fibroblasten erhöhen und/oder verbessern, sind ausgewählt aus Ascorbinsäure, den Estern der Ascorbinsäure mit anorganischen und/oder organischen Säuren, den Ethern der Ascorbinsäure mit Mono-, Oligo- und Polysacchariden, sowie den physiologisch verträglichen Salze dieser Komponenten. Bei den physiologisch verträglichen Salzen sind insbesondere die Zink-, Kupfer- und Mangansalze und die Salze der Alkali- und der Erdalkalimetalle bevorzugt, besonders die Natrium-, Kalium-, Magnesium- und Calcium-Salze. Erfindungsgemäß besonders bevorzugte kosmetische oder dermatologische Zusammensetzungen sind dadurch gekennzeichnet, dass sie mindestens einen Wirkstoff enthalten, der die Interaktion zwischen der extrazellulären Matrix und den Fibroblasten erhöht und/oder verbessert und der ausgewählt ist aus Ascorbinsäure und den Ascorbinsäurederivaten Natriumascorbylphosphat, Magnesiumascorbylphosphat, Ascorbylpalmitat, Dinatriumascorbylphosphat, Dinatriumascorbylsulfat, Natriumascorbat, Magnesiumascorbat, Ascorbylstearat, Ascorbyldipalmitat, Ascorbylacetat, Kaliumascorbyltocopherylphosphat, Chitosanascorbat und Ascorbylglucosid.

Weitere erfindungsgemäß bevorzugte Wirkstoffe, die die Interaktion zwischen der extrazellulären Matrix und den Fibroblasten erhöhen und/oder verbessern, sind ausgewählt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,001 - 2 Gew.-%, bevorzugt 0,01 - 1,6 Gew.-% und besonders bevorzugt 0,03 - 1 Gew.-%, jeweils bezogen auf den Gehalt an Aktivsubstanz in der gesamten Zusammensetzung, enthalten.

Weitere erfindungsgemäß bevorzugte Wirkstoffe, die die Interaktion zwischen der extrazellulären Matrix und den Fibroblasten erhöhen und/oder verbessern, sind ausgewählt aus Lotuskeim-Extrakten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus dem Dipeptid L-Citrullyl-L-arginin und seinen mit mindestens einer, bevorzugt linearen, C₂-C₂₂-Fettsäure N-acylierten und/oder veresterten Derivaten, insbesondere dem N-acetylierten Dipeptidderivat mit der INCI-Bezeichnung Acetyl Citrull Amido Arginine. Eine erfindungsgemäß besonders bevorzugte Zubereitung von Acetyl Citrull Amido Arginine ist unter der Handelsbezeichnung Exsy-Algine von der Firma Exsymol erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Rotweinextrakten (Wine Extract). Ein erfindungsgemäß besonders bevorzugter Rotweinextrakt ist unter der Handelsbezeichnung Sepivinol R von der Firma Seppic erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Traubenkernextrakten (Vitis Vinifera (Grape) Seed Extract), die besonders bevorzugt aus der Chardonnay-Traube stammen. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Extrakten aus Schwarzen Holunderblüten (Sambucus Nigra Flower Extract). Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Weitere erfindungsgemäß bevorzugte Wirkstoffe, die die Interaktion zwischen der extrazellulären Matrix und den Fibroblasten erhöhen und/oder verbessern, sind ausgewählt aus Hydroxystilbenen und deren Estern, insbesondere Resveratrol (trans-Stilben-3,4'-5-triol) und/oder Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Weitere erfindungsgemäß bevorzugte Wirkstoffe, die die Interaktion zwischen der extrazellulären Matrix und den Fibroblasten erhöhen und/oder verbessern, sind ausgewählt aus Keratinhydrolysaten, insbesondere Wollkeratinhydrolysaten. Ein erfindungsgemäß besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf.

Weitere erfindungsgemäß bevorzugte Wirkstoffe, die die Interaktion zwischen der extrazellulären Matrix und den Fibroblasten erhöhen und/oder verbessern, sind ausgewählt aus Conchiolinhydrolysaten. Erfindungsgemäß besonders bevorzugte Conchiolinhydrolysate sind unter den Bezeichnungen Pearl Protein Extract und Pearl Protein Extract BG von der Firma Maruzen erhältlich. Bei Conchiolinhydrolysaten handelt es sich, im Unterschied zu anderen auf dem Markt erhältlichen Perlenextrakten, nicht um Extrakte aus gemahlenen Perlen, wie sie z. B. unter der Bezeichung Crodarom Pearl mit der INCI-Bezeichnung Aqua (Water), Glycerin, Pearl Powder, Maris Sal (Sea Salt) von Croda erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.

Ein weiterer erfindungsgemäß bevorzugter Wirkstoff, der die Interaktion zwischen der extrazellulären Matrix und den Fibroblasten erhöht und/oder verbessert, ist das Peptidderivat L-Glutamylaminoethyl-indol (erhältlich z. B. unter dem Handelsnamen Glistin von der Firma Exsymol).

Weitere erfindungsgemäß bevorzugte Wirkstoffe, die die Interaktion zwischen der extrazellulären Matrix und den Fibroblasten erhöhen und/oder verbessern, sind ausgewählt aus Isoflavonoiden und Isoflavonoid-reichen Pflanzenextrakten.
Zu den Isoflavonoiden zählen erfindungsgemäß die Isoflavone und die Isoflavon-Glycoside. Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Weiterhin ist es erfindungsgemäß bevorzugt, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, insbesondere aus den Sojakeimen, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Lipobelle Soyaglycone (Mibelle AG Cosmetics), Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte kosmetische oder dermatologische Zusammensetzungen sind dadurch gekennzeichnet, dass sie mindestens ein Isoflavonoid in Gesamtmengen von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

Ein weiterer erfindungsgemäß bevorzugter Wirkstoff, der die Interaktion zwischen der extrazellulären Matrix und den Fibroblasten erhöht und/oder verbessert, ist Dihydroquercetin (3,3',4',5,7-Pentahydroxyflavanon), das auch als Taxifolin bezeichnet wird.

Erfindungsgemäß besonders bevorzugte kosmetische oder dermatologische Zusammensetzungen sind dadurch gekennzeichnet, dass sie mindestens einen Wirkstoff, der die Interaktion zwischen der extrazellulären Matrix und den Fibroblasten erhöht und/oder verbessert, in einer Gesamtmenge von 0,000001 - 10 Gew.-%, bevorzugt 0,00001 - 5 Gew.-%, besonders bevorzugt 0,0001 - 2 Gew.-% und außerordentlich bevorzugt 0,005 - 0,5 oder 1 Gew.-%, enthalten, jeweils bezogen auf den Gehalt an Aktivsubstanz in der gesamten Zusammensetzung.

Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen kosmetischen Wirkstoff enthalten, der ausgewählt ist aus :
- Monomeren und Oligomeren von Aminosäuren, N-C₂-C₂₄-Acylaminosäuren, den Estern und/oder den physiologisch verträglichen Metallsalzen dieser Substanzen,
- DNA-Reparaturenzymen,
- DNA- oder RNA-Oligonucleotiden,
- Vitaminen, Provitaminen und Vitaminvorstufen der Gruppen B, E, H und K und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Silymarin,
- Ectoin,
- anorganischen und organischen UV-Filtersubstanzen,
- selbstbräunenden,
- hautberuhigenden,
- feuchtigkeitsspendenden und
- sebumregulierenden Wirkstoffen sowie
- Mischungen dieser Wirkstoffe.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Desmosin, Glutamin, Glutaminsäure, Glycin, Histidin, Homophenylalanin, Hydroxylysin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Taurin, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Erfindungsgemäß bevorzugte, gegebenenfalls N-acylierte und/oder veresterte Dipeptide sind Val-Trp (Dipeptide-2), Asn-Phe, Asp-Phe, N-Palmitoyl-β-Ala-His, Carnosin (β-Ala-His) und N-Palmitoyl-Pro-Arg. Erfindungsgemäß besonders bevorzugte, gegebenenfalls N-acylierte und/oder veresterte Tripeptide sind Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn und N-Acetyl-Arg-Lys-Arg-NH₂. Erfindungsgemäß bevorzugte, gegebenenfalls N-acylierte und/oder veresterte Pentapeptide N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Val-Val-Arg-Pro-Pro-Pro, Ala-Arg-His-Methylnorleucin-Homophenylalanin-Trp (Hexapeptide-7) und Hexapeptide-11 (z. B. Peptamide-6 von Arch Personal Care). Ein erfindungsgemäß besonders bevorzugtes Pentadecapeptid ist z. B. der Rohstoff Vinci 01 von Vincience (Pentadecapeptide-1).

Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus A. *nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten.
T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosomes^{™} (INCI-Bezeichnung: Water, Lecithine, Plankton Extract) mit einer Proteinkonzentration von 0,25 - 2 mg/ml, liposomenverkapselte T4N5 z. B. unter der Bezeichnung Ultrasomes^{™} (INCI-Bezeichnung: Water, Lecithine, Micrococcus Luteus Extract) mit einer Proteinkonzentration von 2 - 5 mg/ml (gemessen mit dem Bradford-Assay) von AGI Dermatics, USA, erhältlich. Weitere erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen kosmetischen Wirkstoff, der ausgewählt ist aus Photosomes^{™} und/oder Ultrasomes^{™}, in Gesamtmengen von 0,1 - 10 Gew.-%, bevorzugt 0,5 - 5,0 Gew.-% und besonders bevorzugt 1,0 - 4,0 Gew.-%, bezogen auf das gesamte Mittel, enthalten.
Weitere erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen kosmetischen Wirkstoff, der ausgewählt ist aus den Monomeren und Oligomeren von Aminosäuren, N-C₂-C₂₄-Acylaminosäuren und/oder den Estern und/oder den physiologisch verträglichen Metallsalzen dieser Substanzen, in Gesamtmengen von 0,00001 - 10 Gew.-%, bevorzugt 0,001 - 5 Gew.-% und besonders bevorzugt 0,005 - 3 Gew.-%, jeweils bezogen auf die gesamte Zusammensetzung, enthalten.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Erfindungsgemäß besonders bevorzugte kosmetische oder dermatologische Öl-in-Wasser-Emulsionen zur topischen Behandlung der Haut enthalten, sind dadurch gekennzeichnet, dass sie weiterhin mindestens einen kosmetischen Wirkstoff enthalten, der ausgewählt ist aus DNA-Oligonucleotiden und RNA-Oligonucleotiden.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glycosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).
Ein erfindungsgemäß besonders bevorzugtes Oligonucleotid ist das Thymidin-Dinucleotid. In den besonders bevorzugten erfindungsgemäßen Zusammensetzungen sind die DNA-Oligonucleotide oder RNA-Oligonucleotide in Mengen von 0,00001 - 5 Gew.-%, bevorzugt 0,0001 -1,0 Gew.-% und besonders bevorzugt 0,0005 - 0,5 Gew.-%, bezogen auf die gesamte Zusammensetzung, enthalten.

Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen kosmetischen Wirkstoff enthalten, der ausgewählt ist aus mindestens einem Vitamin, Provitamin oder einer als Vitaminvorstufe bezeichnete Verbindung aus den Vitamingruppen B, E, H und K und den Estern der vorgenannten Substanzen.
Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-1) eingesetzt werden.

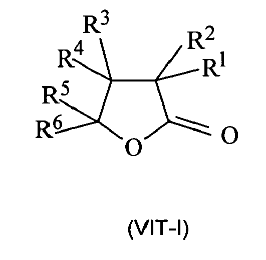

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono- , Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-y-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen kosmetischen Wirkstoff enthalten, der ausgewählt ist aus mindestens einer α-Hydroxycarbonsäure, α-Ketocarbonsäure oder β-Hydroxycarbonsäure oder deren Ester-, Lacton- und/ oder Salzform.
Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.
Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Erfindungsgemäß besonders bevorzugte kosmetische oder dermatologische Öl-in-Wasser-Emulsionen zur topischen Behandlung der Haut sind dadurch gekennzeichnet, dass sie weiterhin Ectoin enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Die erfindungsgemäßen Öl-in-Wasser-Emulsionen sind aufgrund der Möglichkeit, auf übliche Konservierungsmittel zu verzichten, hervorragend als Basis für UV-Filter-haltige Lichtschutzemulsionen geeignet. Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens eine anorganische und/oder mindestens eine organische UV-Filtersubstanz enthalten.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.
Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/ Dimethylsiloxan-Copolymer mit der INCI-Bezeichnung Dimethicodiethylbenzal malonate (CAS-Nr. 207574-74-1, Parsol^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (INCI: Bis-Ethylhexyloxyphenol Methoxyphenyl Triazin, unter dem Namen Tinosorb S bei CIBA erhältlich), Dioctylbutylamidotriazon (INCI: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsil-oxy-silylpropyloxy)-2-hydroxy]-phenyl}-6-(4-methoxyphenyl)-1,3,5-triazin, 2,4-Bis-{[4-(2-methylpro-penyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalydene Dicampher Sulfonic Acid (CAS.-Nr.: 90457-82-2, als Mexoryl SX von der Firma Chimex erhältlich).
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Aber auch anorganische Materialien, insbesondere Silica oder Kieselsäuren, sind als Coatingmittel bevorzugt. Besonders bevorzugte Nanopigmente sind Titandioxid und Zinkoxid.
Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten. Erfindungsgemäß sind die anorganischen UV-Filtersubstanzen bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weitere erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin, besonders bevorzugt aus Kombinationen von Dihydroxyaceton und Erythrulose.
Erfindungsgemäß bevorzugt ist mindestens ein selbstbräunender Wirkstoff in einer Gesamtmenge von 0,01 - 15 Gew.-%, besonders bevorzugt 0,1 - 10 Gew.-%, außerordentlich bevorzugt 1,0 - 5 Gew.-% und weiterhin außerordentlich bevorzugt 2,0 - 4,0 Gew.-%, jeweils bezogen auf die gesamte Zusammensetzung, enthalten.

Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen hautberuhigenden Wirkstoff enthalten. Erfindungsgemäß bevorzugte hautberuhigende Wirkstoffe sind ausgewählt aus Allantoin, α-Bisabolol, α-Liponsäure, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter dem Handelsnamen Calmsphere von Soliance erhältlich ist, Mischungen aus Getreidewachsen, Extrakten aus Schibutter und Argania Spinosa-Öl mit der INCI-Bezeichnung "Spent grain wax and Butyrospermum Parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter dem Handelsnamen Rooibos Herbasec MPE von der Firma Cosmetochem erhältlich sind, Hefeextrakten, besonders bevorzugt das Handelsprodukt Drieline (INCI-Bezeichnung "Sorbitol, Yeast Extract"), erhältlich von der Firma Lanatech, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, sowie beliebigen Mischungen dieser Substanzen.
Der mindestens eine hautberuhigende Wirkstoff ist bevorzugt in einer Gesamtmenge von 0,001 - 5 Gew.-%, besonders bevorzugt 0,01 - 2 Gew.-% und außerordentlich bevorzugt 0,1 - 1 Gew.-%, jeweils bezogen auf die gesamte Zusammensetzung, enthalten.

Weitere erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen feuchtigkeitsspendenden Wirkstoff (F) enthalten, der verschieden ist von den beanspruchten Komponenten a) - f) gemäß Anspruch 1. Erfindungsgemäß bevorzugte zusätzliche feuchtigkeitsspendende Wirkstoffe (F) sind ausgewählt aus Desoxyzuckern, besonders bevorzugt Rhamnose und Fucose, Polysacchariden, die mindestens einen Desoxyzucker-Baustein enthalten, besonders bevorzugt Biosaccharide Gum-1, enthalten beispielsweise in dem Handelsprodukt Fucogel^{®} von Solabia Biosaccharide Gum-2, enthalten beispielsweise in dem Handelsprodukt Rhamnosoft^{®} von Solabia, Biosaccharide Gum-3, enthalten beispielsweise in dem Handelsprodukt Fucogenol^{®} von Solabia, Biosaccharide Gum-4, enthalten beispielsweise in dem Handelsprodukt Glycofilm^{®} von Solabia, Mischungen aus Biosaccharide Gum-2 und Biosaccharide Gum-3, beispielsweise erhältlich als Handelsprodukt Elastinol plus^{®} von Solabia, weiterhin Harnstoff, Bis-N,N'-(2-Hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat, Chondroitin-6-sulfat, Kombinationen von Bis-N,N'-(2-Hydroxyethyl)harnstoff und Harnstoff und besonders bevorzugt Kombinationen von Bis-N,N'-(2-Hydroxyethyl)harnstoff, Harnstoff und Milchsäure(salzen).

Der mindestens eine zusätzliche feuchtigkeitsspendende Wirkstoff (F), der verschieden ist von den beanspruchten Komponenten a) - f) gemäß Anspruch 1, ist bevorzugt in einer Gesamtmenge von 0,001 - 10 Gew.%, besonders bevorzugt 0,01 - 5 Gew.-% und außerordentlich bevorzugt 0,1 - 1 oder 2 Gew.-%, jeweils bezogen auf die gesamte Zusammensetzung, enthalten.

Weitere erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen sebumregulierenden Wirkstoff enthalten. Derartige Wirkstoffe entfalten in den erfindungsgemäßen Öl-in-Wasser-Emulsionen eine unerwartet hohe Wirkung gegen Akne und/oder unreine Haut.
Erfindungsgemäß bevorzugte sebumregulierende Wirkstoffe sind ausgewählt aus Azelainsäure, Sebacinsäure, 10-Hydroxydecansäure, 1,10-Decandiol, die als erfindungsgemäß besonders bevorzugte Dreierkombination in dem Handelsprodukt Acnacidol PG von Vincience eingesetzt werden, weiterhin aus dem Handelsprodukt Azeloglicina (Potassium Azeloyl Diglycinate) von Sinerga, Extrakten aus Spiraea Ulmaria, wie sie z. B. im Produkt Seboregul der Firma Silab enthalten sind, weiterhin aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und den Wirkstoffmischungen Asebiol^{®} BT 2 (Laboratoires Sérobioiogiques, INCI: Aqua, Hydrolyzed Yeast Protein, Pyridoxine, Niacinamide, Glycerin, Panthenol, Allantoin, Biotin) und Antifettfaktor^{®} COS-218/2-A (Cosmetochem, INCI: Aqua, Cetyl-PCA, PEG-8 Isolauryl Thioether, PCA, Cetyl Alcohol).
Der mindestens eine sebumregulierende Wirkstoff ist bevorzugt in einer Gesamtmenge von 0,001 - 5 Gew.-%, besonders bevorzugt 0,01 - 2 Gew.-% und außerordentlich bevorzugt 0,1 bis 1 Gew.-%, jeweils bezogen auf die gesamte Zusammensetzung, enthalten.

Erfindungsgemäß besonders bevorzugte kosmetische oder dermatologische Öl-in-Wasser-Emulsionen zur topischen Behandlung der Haut sind dadurch gekennzeichnet, dass sie weiterhin mindestens einen zusätzlichen konditionierenden Wirkstoff enthalten. Dieser zusätzliche konditionierende Wirkstoff ist verschieden von den beanspruchten Komponenten a) - f) gemäß Anspruch 1. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.
Erfindungsgemäß bevorzugte zusätzliche konditionierende Wirkstoffe, die verschieden sind von den beanspruchten Komponenten a) - f), sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, lsoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sind, verzweigten gesättigten oder ein- oder mehrfach ungesättigten, Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin oder 1,2-Propylenglycol, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die nachfolgenden Formulierungsbeispiele sollen den Gegenstand der Erfindung erläutern, ohne ihn hierauf zu beschränken.

Die Zusammensetzungen 1 - 5 sind bevorzugte Ausführungsformen von erfindungsgemäßen pflegenden Tagescremes auf Basis einer Öl-in-Wasser-Emulsion mit selbstkonservierender Wirkung und einem Effekt zur Verbesserung bzw. Behandlung von unreiner Haut.

| | 1 | 2 | 3 | 4 | 5 |
|---|---|---|---|---|---|
| Montanov 202 | 5 | 4 | 3 | 3 | 4 |
| Lanette O | 4 | 5 | 5,5 | 5,5 | 5 |
| Cetiol SN | 2 | 1,5 | 1 | 1 | 1,5 |
| Baysilon M 350 | 1 | 1 | 1 | 1 | 1 |
| Cutina PES | 3 | 2 | 2 | 2 | 2 |
| Glycerin, 86 %ig | 3 | 4 | 5 | 5 | 4 |
| 1,6-Hexandiol | 6 | 5 | 4 | 4 | 5 |
| 1,2-Propylenglycol | 5 | 5 | 5 | 5 | 5 |
| Lipochroman 6 | 0,01 | 0,01 | 0,01 | 0,01 | 0,01 |
| Talkum | 1 | 1 | 1 | 1 | 1 |
| Xanthan-Gum | 0,15 | 0,1 | 0,2 | 0,2 | 0,1 |
| Sepiplus 400 | 1,2 | 1,4 | - | - | 1,4 |
| Simulgel NS | - | - | 1,2 | 1,2 | - |
| Acnacidol PG | - | - | - | 0,5 | - |
| Matrixyl 3000 | - | - | - | - | 1,0 |
| Enteline 2 | - | - | - | - | 0,5 |
| Hydrovance | - | - | - | 4,0 | - |
| Parfum | 0,30 | 0,30 | 0,30 | 0,30 | 0,30 |
| Aqua | ad 100 | ad 100 | ad 100 | ad 100 | ad 100 |

Alle Mengenangaben sind in Gew.-%, bezogen auf die gesamte Zusammensetzung.

### Liste der verwendeten Rohstoffe

| Handelsname | INCI-Bezeichnung | Lieferant/Hersteller |
|---|---|---|
| | | |
| Acnacidol PG | Aqua (Water), Propylene Glycol, Sebacic acid, 10-hydroxydecanoic acid, 1,10-decanediol | Vincience |
| Baysilone-Öl M 350 | Dimethicone | GE Bayer Silicones |
| Biopeptide CL | Glyceryl Polymethacrylate, Propylene Glycol, N-Palmitoyl-Gly-His-Lys | Sederma |
| Biopeptide EL | Glyceryl Polymethacrylate, PEG-8, N-Palmitoyl-Val-Gly-Val-Ala-Pro-Gly | Sederma |
| Calmiskin | Water (and) Mentha piperita (Peppermint) Leaf Extract | Silab |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 | Acrylates/C10-30 Alkyl Acrylate Crosspolymer | Noveon |
| Cetiol B | Dibutyl Adipate | Cognis |
| Cetiol^{®} HE | PEG-7 Glyceryl Cocoate | Cognis |
| Cetiol^{®} PGL | Hexyldecanol, Hexyldecyl Laurate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cosmedia Guar^{®} C 261 | Guar Hydroxypropyl Trimonium Chloride | Cognis |
| Cremophor A 6 | CETEARETH-6, STEARYL ALCOHOL | BASF |
| Crodarom Chardonnay L | Propylene Glycol, Water, Vitis Vinifera (Grape) Seed Extract | Croda |
| Cutina^{®} PES | Pentaerythrityl distearate | Cognis |
| DERMOSOFT OCTIOL | 1,2-Octandiol | Dr. Straetmans |
| Dow Corning^{®}245 Fluid | Cyclomethicone | Dow Corning |
| Dow Corning 9040 | Cyclomethicone/ Dimethicone Crosspolymer | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinyl-pyrrolidon | National Starch and Chemical |
| EDENOR L2 S M | Palmitic Acid, Stearic Acid (ca. 1: 1) | Cognis |
| Ederline H | PEG-40 Hydrogenated Castor Oil, PPG-2-Ceteareth-9, Pyrus Malus (Apple) Fruit Extract | Seporga |
| Ederline L | Hexyldecanol, Pyrus Malus (Apple) Fruit Extract | Seporga |
| Enteline 2 | Butylene Glycol, Glycerine, Hydrolyzed Enteromorpha compressa | BiotechMarine |
| Eumulgin B 2 | Ceteareth-20 | Cognis |
| Eumulgin B 3 | Ceteareth-30 | Cognis |
| Eumulgin 05 | Oleth-5 | Cognis |
| Eusolex^{®} OCR | Octocrylene | Merck KGaA |
| Eutanol G | OCTYLDODECANOL | Cognis |
| Exsy Algine | ACETYL CITRULL AMIDO ARGININE | Exsymol |
| Fucogel 1000 | Biosaccharide Gum-1 | Solabia |
| Germall^{®} 115 | Imidazolidinyl Urea | Sutton Laboratories |
| Glistin | Wasser, L-Glutamylaminoethyl indole | Exsymol |
| Glucamate^{®}DOE 120 | PEG-120 Methyl Glucose Dioleate | AMERCHOL |
| Herbasol Extrakt Capsicum | Propylene Glycol, Aqua (Water), Capsicum Frutescens Fruit Extract, Sorbitol | Cosmetochem |
| Hibiscin^{®} HP-LS-9198 | Water, Hibiscus esculentus Seed Extract, Phenoxyethanol | Laboratoires Sérobiologiques |
| Honeyquat^{®} 50 | Hydroxypropyltrimonium Honey | BROOKS |
| Hotact VBE | Vanillyl Butyl Ether | Takasago |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Keratolite | AQUA (WATER), MALIC ACID, BUTYLENE GLYCOL, PRUNUS AMYGDALUS DULCIS (SWEET ALMOND) FRUIT EXTRACT, SODIUM BENZOATE, PHENOXYETHANOL, METHYLPARABEN,PROPYLPARABEN, ETHYLPARABEN | Coletica |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} O | Cetearyl Alcohol | Cognis |
| LANETTE^{®} 22 | Behenyl Alcohol | Cognis |
| Lipochroman 6 | DIMETHYLMETHOXY CHROMANOL | Lipotec |
| Lipoid S 75-3 | Aqua, Lecithine | Lipoid GmbH |
| Lotus Germ Extract | Water, Butylene Glycol, Nelumbo Nucifera Germ Extract | Maruzen |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov L | C14-22 Alcohols/C12-20 Alkyl Glucoside | Seppic |
| Montanov 202 | ARACHIDYL ALCOHOL, BEHENYL ALCOHOL, ARACHIDYL GLUCOSIDE | Seppic |
| Myritol 318 | Caprylic/Capric Triglyceride | Cognis |
| MYRITOL^{®}312 | Caprylic/Capric Triglyceride | Cognis |
| MYRITOL^{®} PC | PROPYLENE GLYCOL DICAPRYLATE/DICAPRATE | Cognis |
| Natipide II PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Neutrol TE | TETRAHYDROXYPROPYL ETHYLENEDIAMINE | BASF |
| Novata AB | Cocoglycerides | Cognis |
| Ocaline | Sea Water (and) Water (and) Cucurbita pepo (pumpkin) seed extract | Soliance |
| Olea europ. Fol extr. S. sicc. | Olea Europea (Olive) Leaf Extract | Fruitarom |
| Omega-CH-Aktivator | Tripeptide-1 | GfN |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Phenonip^{®} | Phenoxyethanol, Methylparaben, Ethylparaben, Propylparaben, Butylparaben, ca. 28 % Aktivsubstanz | NIPA |
| Photosomes | Aqua, Lecithine, Plancton Extract | AGI Dermatics |
| Phytodermin | SOYBEAN PROTEIN GLYCINE SOJA (Linne) | CLR Chem. Laboratorium Kurt Richter |
| Phytokine | Aqua, Butylene Glycol, HYDROLYZED SOY PROTEIN, Methylparaben, Ethylparaben, Propylparaben | Coletica |
| Ridulisse C | HYDROLYZED SOY PROTEIN | Silab |
| Sambucus AO | Water Glycerin, Sambucus Nigra Flower Extract | Alpaflor/Centerche m |
| Seanergilium BG | Butylene Glicol, Laminaria Digitata Extract, Methylparaben | Coletica |
| Sensiva^{®} SC 50 | 2-Ethylhexylglycerinether | Schülke & Mayr |
| Sepigel^{®}305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepiplus 400 | Polyacrylate-13, POLYISOBUTENE, POLYSORBATE 20, Aqua | Seppic |
| Sepilift DPHP | Dipalmitoyl Hydroxyproline | Seppic |
| Silymarin Phytosome | Silybum Marianum Extract and Phospholipids | Indena SpA |
| Symdiol 68 | 1,2-Hexandiol, 1,2-Octandiol | Symrise |
| Simulgel NS | Aqua n(Water)/Hydroxyethyl Acrylate / Sodium Acryloyldimethyl Taurate Copolymer / Squalane / Polysorbate 60 | Seppic |
| Soy Protein Isolate | | Protein Technology International |
| Stenol 16/18 | Cetearylalkohol | Cognis |
| Symdiol 68 | 1,2-Octandiol, 1,2-Hexandiol | Symrise |
| SYN^{®}-COLL | Water, Palmitoyl-Lys-Val-Lys | Pentapharm |
| Tego Carbomer 140 | Carbomer | Degussa |
| Tioveil^{®}-AQ-N | CI 77891 (Titanium Dioxide),Alumina, Silica, Sodium Polyacrylate | Uniqema (Tioxide Specialties) |
| Ultrasomes | Aqua, Lecithine, Micrococcus Luteus Extract | AGI Dermatics |
| Uvinul MBC 95 | 4-METHYLBENZYLIDENE CAMPHOR | BASF |
| Uvinul MS 40 | BENZOPHENONE-4 | BASF |

## Patentansprüche

1. Kosmetische oder dermatologische Öl-in-Wasser-Emulsion zur topischen Behandlung der Haut, enthaltend
a) mindestens einen linearen, gesättigten primären Alkohol mit 14 - 30 Kohlenstoffatomen,
b) mindestens ein Alkylglycosid mit einem linearen oder verzweigten Alkyl- oder Alkenylrest mit 14 - 30 Kohlenstoffatomen und einem Mono-, Di- oder Triglycosidrest,
c) mindestens einen Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einen Ester aus Pentaerythrit und einer geradkettigen oder verzweigten, gesättigten oder ungesättigten Fettsäure mit 3 - 30 Kohlenstoffatomen,
e) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon,
f) Xanthan-Gum,
**dadurch gekennzeichnet, dass** die Komponenten a:b:c:d:e:f in Mengen von (1,5 - 14 Gew.-%) : (0,1 - 2 Gew.-%) : (0,5 - 5 Gew.-%) : (1 - 7 Gew.-%) : (1 - 25 Gew.-%) : (0,1 - 1 Gew.-%) vorliegen, jeweils bezogen auf das Gesamtgewicht der kosmetischen oder dermatologischen Öl-in-Wasser-Emulsion.

2. Kosmetische oder dermatologische Zusammensetzung gemäß Anspruch 1, **dadurch gekennzeichnet, dass** mindestens ein Antioxidans enthalten ist.

3. Kosmetische oder dermatologische Zusammensetzung gemäß Anspruch 2, **dadurch gekennzeichnet, dass** das mindestens eine Antioxidans ausgewählt ist aus 2,2-Dialkylchromanen, 2,2-Dialkylchromenen, Flavonoiden, Ubichinonen, Ubichinolen, Pentaerythrittetrakis[3(3,5-ditert.-butyl-4-hydroxyphenyl)propionat], Urocaninsäure, Carotinoiden, Ascorbinsäure und ihren Derivaten, insbesondere ihren Estern und Salzen, lsoascorbinsäure und ihren Derivaten, insbesondere ihren Estern und Salzen, Ferulasäure und ihren Estern, insbesondere Ethylferulat, Kaffeesäure, Rosmarinsäure, 2,6-Di-tert.-butyl-4-methylphenol, tert.-Butyl-4-methoxyphenol, sowie Mischungen hiervon.

4. Kosmetische oder dermatologische Zusammensetzung gemäß einem der vorstehenden Ansprüche, **dadurch gekennzeichnet, dass** insgesamt, jeweils bezogen auf das Gesamtgewicht der Öl-in-Wasser-Emulsion, mindestens ein Antioxidans in einer Gesamtmenge von 0,001 - 1 Gew.-%, bevorzugt 0,005 - 0,5 Gew.-%, besonders bevorzugt 0,01 - 0,3 Gew.-% und außerordentlich bevorzugt 0,05 - 0,1 Gew.-%, enthalten ist.

5. Kosmetische oder dermatologische Zusammensetzung gemäß einem der vorstehenden Ansprüche, **dadurch gekennzeichnet, dass** insgesamt, jeweils bezogen auf das Gesamtgewicht der Öl-in-Wasser-Emulsion, maximal 0,3 Gew.-%, bevorzugt maximal 0,2 Gew.-% und besonders bevorzugt maximal 0,1 Gew.-%, an Konservierungsmitteln enthalten ist.

6. Kosmetische oder dermatologische Zusammensetzung gemäß einem der vorstehenden Ansprüche, **dadurch gekennzeichnet, dass** sie frei ist von Konservierungsmitteln.

7. Kosmetische oder dermatologische Zusammensetzung gemäß einem der vorstehenden Ansprüche, enthaltend mindestens einen weiteren kosmetischen Wirkstoff, ausgewählt aus:
a) Wirkstoffen, die die Kollagensynthese stimulieren,
b) Wirkstoffe, die die Interaktion zwischen der extrazellulären Matrix und den Fibroblasten erhöht und/oder verbessern,
c) Monomeren und Oligomeren von Aminosäuren, N-C₂-C₂₄-Acylaminosäuren, den Estern und/oder den physiologisch verträglichen Metallsalzen dieser Substanzen,
d) DNA-Reparaturenzymen,
e) DNA- oder RNA-Oligonucleotiden,
f) Vitaminen, Provitaminen und Vitaminvorstufen der Gruppen B, E, H und K und den Estern der vorgenannten Substanzen,
g) α-Hydroxycarbonsäuren, α-Ketocarbonsäuren, β-Hydroxycarbonsäuren und deren Ester-, Lacton- und/oder Salzform,
h) Flavonoiden und Flavonoid-reichen Pflanzenextrakten, die nicht die Degranulierung von Mastzellen und/oder die Ausschüttung von Histamin reduzieren können,
i) Ubichinon und Ubichinol sowie deren Derivaten,
j) Silymarin,
k) Ectoin,
l) anorganischen und organischen UV-Filtersubstanzen,
m) selbstbräunenden Wirkstoffen,
n) hautberuhigenden Wirkstoffen,
o) feuchtigkeitsspendenden Wirkstoffen,
p) sebumregulierenden Wirkstoffen,
q) sowie Mischungen dieser Wirkstoffe a) - q).

8. Verwendung einer Kombination aus
a) mindestens einem linearen, gesättigten primären Alkohol mit 14 - 30 Kohlenstoffatomen,
b) mindestens einem Alkylglycosid mit einem linearen oder verzweigten Alkyl- oder Alkenylrest mit 14 - 30 Kohlenstoffatomen und einem Mono-, Di- oder Triglycosidrest,
c) mindestens einem Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einem Ester aus Pentaerythrit und einer linearen oder verzweigten, gesättigten oder ungesättigten Fettsäure mit 3 - 30 Kohlenstoffatomen,
e) mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%) : (0,1 -2 Gew.-%) : (0,5 - 5 Gew.-%) : (1 - 7 Gew.-%) : (1 - 25 Gew.-%) : (0,1 - 1 Gew.-%), jeweils bezogen auf das Gesamtgewicht der kosmetischen oder dermatologischen Öl-in-Wasser-Emulsion, vorliegen, zur Herstellung einer konservierungsmittelfreien kosmetischen oder dermatologischen Öl-in-Wasser-Emulsion.

9. Verwendung einer Kombination aus
a) mindestens einem linearen, gesättigten primären Alkohol mit 14 - 30 Kohlenstoffatomen,
b) mindestens einem Alkylglycosid mit einem linearen oder verzweigten Alkyl- oder Alkenylrest mit 14 - 30 Kohlenstoffatomen und einem Mono-, Di- oder Triglycosidrest,
c) mindestens einem Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einem Ester aus Pentaerythrit und einer linearen oder verzweigten, gesättigten oder ungesättigten Fettsäure mit 3 - 30 Kohlenstoffatomen,
e) mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%) : (0,1 - 2 Gew.-%) : (0,5 - 5 Gew.-%) : (1 - 7 Gew.-%) : (1 - 25 Gew.-%) : (0,1 - 1 Gew.-%), jeweils bezogen auf das Gesamtgewicht der kosmetischen oder dermatologischen Öl-in-Wasser-Emulsion, vorliegen, zur Herstellung einer kosmetischen oder dermatologischen Öl-in-Wasser-Emulsion, die das Wachstum von Propionibacterium acnes inhibiert.

10. Verwendung einer Kombination aus
a) mindestens einem linearen, gesättigten primären Alkohol mit 14 - 30 Kohlenstoffatomen,
b) mindestens einem Alkylglycosid mit einem linearen oder verzweigten Alkyl- oder Alkenylrest mit 14 - 30 Kohlenstoffatomen und einem Mono-, Di- oder Triglycosidrest,
c) mindestens einem Ester aus einem geradkettigen oder verzweigten Alkanol mit 1 bis 30 Kohlenstoffatomen, das nicht Pentaerythrit ist, und einer geradkettigen oder verzweigten Fettsäure mit 3 bis 18 Kohlenstoffatomen,
d) mindestens einem Ester aus Pentaerythrit und einer linearen oder verzweigten, gesättigten oder ungesättigten Fettsäure mit 3 - 30 Kohlenstoffatomen,
e) mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon,
f) Xanthan-Gum,
wobei die Komponenten a:b:c:d:e:f in Mengen von (1,5 - 14 Gew.-%) : (0,1 - 2 Gew.-%) : (0,5 - 5 Gew.-%) : (1 - 7 Gew.-%) : (1 - 25 Gew.-%) : (0,1 - 1 Gew.-%), jeweils bezogen auf das Gesamtgewicht der kosmetischen oder dermatologischen Öl-in-Wasser-Emulsion, vorliegen, zur Herstellung einer kosmetischen oder dermatologischen Öl-in-Wasser-Emulsion zur Behandlung von unreiner Haut und/oder Akne.
